# EUROPÄISCHE PATENTSCHRIFT

(11) **EP 0 492 499 B1**
(45) Veröffentlichungstag und Bekanntmachung des Hinweises auf die Patenterteilung: **20.11.1996**
(21) Anmeldenummer: 91121935.0
(22) Anmeldetag: 20.12.1991
(51) Int. Cl.: G01N 33/546, G01N 33/58, G01N 33/76

(54) **Verfahren zur Bestimmung eines Analyten**
Method for the determination of an analyte
Procédé pour la détermination d'une analyte

(30) Priorität: 21.12.1990 DE 4041080
(43) Veröffentlichungstag der Anmeldung: 01.07.1992
(73) Patentinhaber: BEHRINGWERKE Aktiengesellschaft, 35001 Marburg (DE)
(72) Erfinder: Wissel, Thomas, Dr., W-3550 Marburg (DE)

(56) Entgegenhaltungen:
- EP-A- 0 038 181
- EP-A- 0 331 127
- EP-A- 0 488 152
- DE-A- 3 842 125
- US-A- 4 576 912
- US-A- 4 687 734
- CLINICAL CHEMISTRY, Band 29, Nr. 8, August 1983, Washington, DC (US); WEEKS et al., Seiten 1474-1479

## Beschreibung

Die Erfindung betrifft analytische Methoden, in denen die Verteilung einer markierten Substanz zwischen einer flüssigen und einer Festphase bestimmt wird. Die Assays schließen Festphasenreagenzien ein, die partikulär oder monolithisch, wie z. B. ein beschichtetes Röhrchen, sein können. Assays dieser Art sind dem Fachmann an sich bekannt und schließen Immunoassays und immunometrische Assays ein.

In vielen Fällen wird nach der Inkubation der Reagenzien die Festphase von der flüssigen Phase abgetrennt, sogenannte heterogene oder festphasen-immunometrische Bestimmungen. Eine partikuläre Festphase kann durch Sedimentation, Zentrifugation, Filtration oder ein magnetisches Feld von der flüssigen Phase abgetrennt werden.

Festphasen zur Verwendung in solchen Immunoassays sind ebenfalls dem Fachmann bekannt und in der Literatur beschrieben, siehe z. B. VOLLER, A. et al., Bull.World Health Org. 53, 55 - 65 (1976).

Magnetisierbare Partikel, die suspendierbar, aber unlöslich in der flüssigen Phase sind, haben den Vorteil, daß sie schnell von der flüssigen Phase in einem Magnetfeld abgetrennt werden können.

Verfahren zur Bestimmung von Analyten unter Anwendung von magnetischen Partikeln sind z. B. in den US Patenten 4,554,088, 3,933,997 sowie in der europäischen Patentanmeldung EP 0 136 126 beschrieben. In diesen Verfahren werden mit Radioisotopen markierte Substanzen zur Bestimmung eines Analyten eingesetzt.

Diese oben beschriebenen Verfahren haben den entscheidenden Nachteil, daß sie auf die Verwendung von Radioisotopen mit ihrem bekannten Gefahrenpotential und der daher erschwerten Handhabung und Abfallbeseitigung beschränkt sind.

Als Alternative zur Verwendung von Radioisotopen als Markierung (Label) haben Enzyme und damit die Enzymimmunoassays breite Anwendung gefunden. In diesem Verfahren wird das zu markierende Reagenz mit einem Enzym verknüpft und nach der Ausbildung des Festphasenrezeptor-Analyt-Komplexes oder des Festphasenrezeptor-Analyt-Rezeptor-Komplexes wird eine Inkubation mit einem Substrat des Enzyms durchgeführt. Die Enzymreaktion erzeugt nun ein gefärbtes, lumineszierendes oder fluoreszierendes Derivat des Substrats. Es wird nach Abschluß der Reaktion statt der Radioaktivität die Absorption, Lumineszenz oder Fluoreszenz gemessen.

Geeignete magnetisch abtrennbare Partikel weisen durch ihren Gehalt an z. B. Eisenoxydpartikeln eine Eigenfärbung auf. Sie absorbieren in Abhängigkeit ihrer Größe und ihres Magnetitgehalts Licht. Diese Eigenabsorption der Partikel verringert unspezifisch das Signal einer Absorptions-, Lumineszenz- oder Fluoreszenzmessung. Das Ausmaß der Lichtabsorption hängt u. a. von der eingesetzten Magnetpartikelmenge ab.

Die Abhängigkeiten gelten naturgemäß auch, wenn auch in unterschiedlichem Maße, für nicht magnetisch anziehbare Teilchen.

Die Patentanmeldung EP 0 177 191 beschreibt einen heterogenen Enzymimmunoassay mit Magnetpartikeln als Festphase, einem Enzym-Analyt-Konjugat und einem chromogenen Substrat als signalgebendes Reagenz. Die Magnetpartikel müssen vor der Messung der Absorption abgetrennt werden. Der Nachteil dieses Verfahrens liegt in der Notwendigkeit eines zusätzlichen weiteren Inkubationsschrittes von üblicherweise 20 - 60 min zur Erzeugung des Signals. Das Verfahren benötigt also einen erheblich größeren Zeit-und Arbeitsaufwand als die vergleichbaren Verfahren, die Radioisotope verwenden. Ein weiterer Nachteil liegt darin begründet, daß dies Verfahren nicht bei heterogenen Immunoassays, die ein direktes chemilumineszierendes oder fluoreszierendes Label verwenden, angewendet werden kann.

In enzymverstärkten Lumineszenz-Systemen, wie sie in der Literatur bekannt sind und z.B. in der Patentanmeldung EP 0 149 565 eingesetzt werden, ist dies nicht möglich. Dort wird eine Peroxidase benutzt, um ein Substrat vom Luminol-Typ zur Lichtaussendung anzuregen. Werden in diesem Verfahren die Partikel, die den Komplex aus Rezeptor und Enzym tragen, abgetrennt, kann keine Chemilumineszenz mehr erzeugt und damit auch kein Signal gemessen werden.

Aus US 4,576,912 ist ein Verfahren bekannt, bei dem ein Fluorophor über ein Carriermolekül an den spezifischen Bindungspartner gekoppelt ist. Der Fluorophor wird nach der Abtrennung des ungebundenen Konjugats durch chemische oder enzymatische Hydrolyse des Carriermoleküls freigesetzt, diese Hydrolyse dauert typischerweise 60 min. Chemilumineszente Label werden in dem Patent nicht in Erwägung gezogen.

In der älteren EP 0 488 152 werden Immunoassays beschrieben, in denen der Analyt über Nucleinsäuren an die Fesphase gebunden wird und das Markierungsreagenz durch ein Freisetzungsreagenz vor der Messung abgelöst wird.

In der DE 38 42 125 wird ein Bestimmungsverfahren beschrieben, in dem die Markierung, ein inerter Partikel, nach der Freisetzung mittels photoakustischer Spektroskopie bestimmt wird.

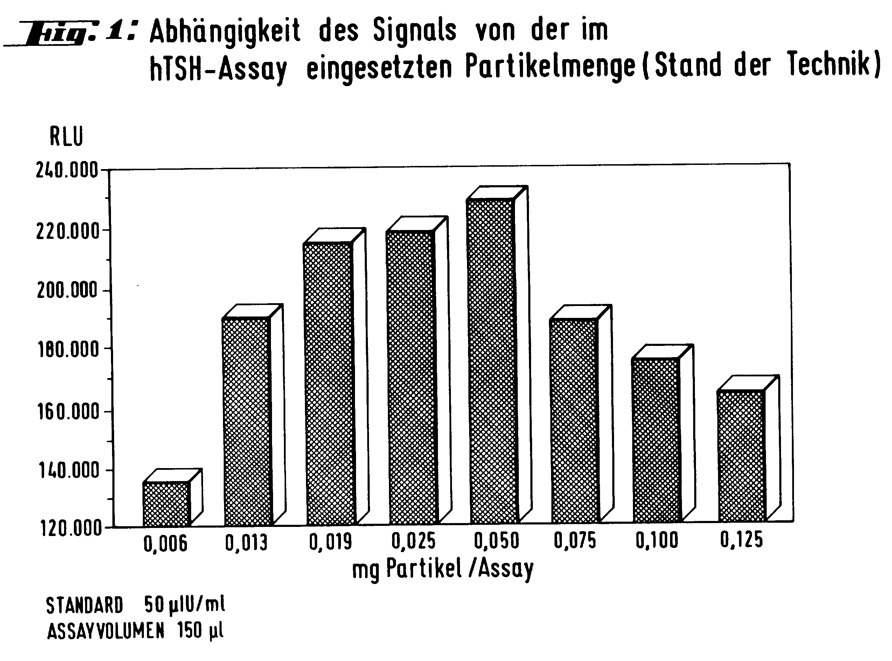

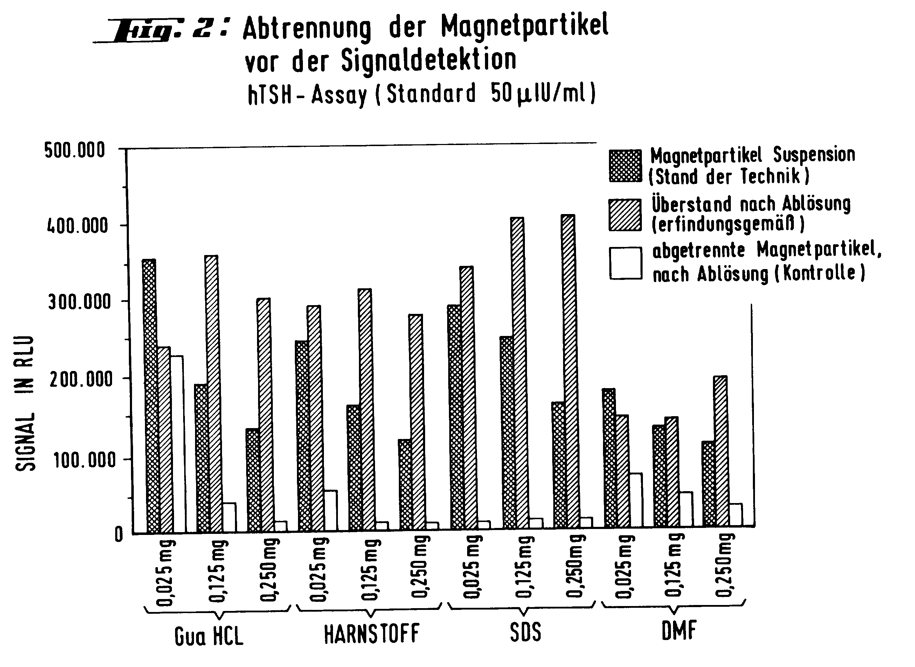

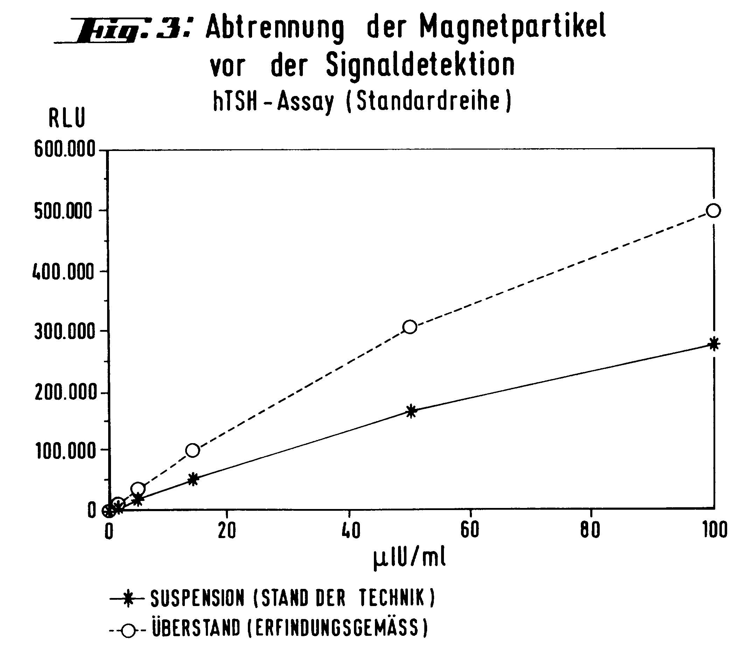

Den Stand der Technik stellt das Verfahren dar, das in der EP 0 149 565 beschrieben wird. In dieser Patentanmeldung wird die Messung von Chemilumineszenz und Fluoreszenz in Gegenwart von suspendierten Magnetpartikeln beschrieben. Es wird dort gezeigt, das es einen schmalen Konzentrationsbereich für die Magnetpartikel gibt, in dem trotz der Lichtabsorption die Durchführung eines Assays möglich gemacht wird (siehe auch Fig. 1). Damit werden aber, wie die vorliegende Erfindung zeigt, die Vorteile der Magnetpartikeltechnologie, wie große Oberfläche und kurze Diffusionswege nicht ausgeschöpft.

Überraschenderweise wurde nun gefunden, daß es durch die Zugabe von geeigneten Substanzen möglich ist, durch die Ablösung des Labels und nach Abtrennung der Festphase das Signal der markierten Reagenzien ungestört zu messen. Das gemessene Signal zeigt eine strenge Relation zur Konzentration des eingesetzten Analyten.

Durch das erfindungsgemäße Verfahren wird eine deutlich erhöhte Signalausbeute und eine bessere Signaldynamik erreicht, als nach dem in EP 0 149 565 beschrieben Verfahren (siehe auch Fig. 2).

Ohne damit einen bestimmten Wirkungsmechanismus festlegen zu wollen, gehen wir davon aus, daß die erfindungsgemäßen Substanzen den die Markierung tragenden Komplex mit der Festphase spalten und die Markierung in einer geeigneten Form freisetzen.

Durch die vorliegende Erfindung wird es nun möglich, die Festphase in hohen Konzentrationen einzusetzen. Durch die vergrößerte Oberfläche wird es auch möglich, den Festphasenrezeptor in einem größeren Überschuß gegenüber dem Analyten einzusetzen. Darüberhinaus werden die Diffusionswege wesentlich verkürzt. Durch beide Effekte wird die Lage des Gleichgewichts sowie die Reaktionskinetik eines Assays günstig beeinflußt.

Die vorliegende Erfindung betrifft nun ein heterogenes, immunchemisches Verfahren zur Bestimmung eines Analyten in einem flüssigem Medium, welches die Verfahrensschritte gemäß den vorliegenden Ansprüchen enthält.

Zwischen den einzelnen Schritten : des Verfahrens gemäß Anspruch 1 können gegebenenfalls Waschschritte eingefügt werden.

Das Verfahren ist grundsätzlich für Systeme geeignet, mit einem fluoreszenten oder chemilumineszenten Label, bevorzugt ist dabei eine chemilumineszente Markierung.

In der Literatur werden verschiedene Chemilumineszenzsysteme beschrieben, darunter haben Luminol, Isoluminol und Acridiniumderivate größere Bedeutung erlangt. Die Luminol und Isoluminolderivate sind bei H.R. Schroeder et al. " Methods in Enzymology" Academic Press Inc, New York, Vol LVII 424ff. beschrieben. Die Anwendung der Acridiniumderivate als Markierungssubstanzen ist bei Weeks et al. (Clin.Chem 29/8, 1983, 1474-1479) beschrieben. Die Chemilumineszenz von Acridiniumderivaten kann durch Zugabe von alkalischer H₂O₂ gestartet und innerhalb weniger Sekunden gemessen werden.

Diese Verbindungen besitzen als Markierungsreagenz den Vorteil, daß zur Signalauslösung, im Gegensatz zu den enzymverstärkten Lumineszenz Systemen, kein weiterer Inkubationsschritt nötig ist. Darüberhinaus sind die Acridiniumverbindungen gegenüber den hier verwendeten Reagenzien stabil.

Bevorzugt als chemilumineszente Label sind die in der EP 0 257 541 und EP 0 330 050 beschriebenen AcridiniumVerbindungen.

Der genaue Aufbau der hier verwendeten magnetisch anziehbaren Partikel ist dem Fachmann an sich bekannt und in Bezug auf die Erfindung weitgehend ohne Bedeutung, bevorzugt sind superparamagnetische Teilchen, die vorteilhafterweise als Kompositpartikel oder reine Magnetitteilchen ausgeführt sein können.
Grundsätzlich können auch nicht magnetisierbare Partikel eingesetzt werden, wobei dann das Trennverfahren dem jeweiligen Partikel angepaßt sein muß, z. B. Zentrifugation oder Chromatographie. Auch solche partikulären Festphasen sind dem Fachmann bekannt, wie z. B. Latexpartikel, die gefärbt oder ungefärbt sein können.

Die Partikel haben bevorzugterweise eine Größe von 0,1 - 5,0 µm, besonders bevorzugt ist ein Bereich von 0,2 - 2,5 µm, ganz besonders bevorzugt von 0,2 - 1,0 µm.

Vorteilhafterweise werden in einem Assay kleine Partikel eingesetzt, die bei gleicher Partikelgesamtmasse eine große Anzahl und eine insgesamt große Oberfläche aufweisen. Durch eine hohe Anzahl von Partikeln wird der Abstand von Partikel zu Partikel in einem gegebenen Volumen sehr klein, daraus folgt, daß die Diffusionswege der gelösten Analyten und der markierten Substanz zur Partikeloberfläche sehr klein werden. Dementsprechend wird der Reaktionsablauf zur Ausbildung der Festphasen gebundenen Rezeptorkomplexe beschleunigt. Außerdem kann durch die größere gesamte Festphasenoberfläche eine erhöhte Rezeptorkonzentration erreicht werden. Hohe Rezeptorkonzentrationen führen zu einer hohen Anbindung des Analyten und ebenfalls zu einer Beschleunigung der Reaktion.
Kleine magnetisch abtrennbare Kompositpartikel benötigen allerdings eine hohen Anteil an magnetisierbarem Material, um in praktikablen Zeiten von der Reaktionslösung abgetrennt werden zu können.
Die Lichtabsorption einer Partikelsuspension ist u. a. von der Art, von der Größe, dem Gehalt an magnetischem Material und von der Konzentration der eingesetzten Partikel abhängig. Wird, wie im Stand der Technik beschrieben, das Signal in Gegenwart der suspendierten Partikel gemessen, dann durchläuft das meßbare Signal in einem Assay zur Bestimmung von z. B. TSH in Serum mit steigender Partikelmenge pro Ansatz ein Maximum. Mit Überschreiten der unter diesen Bedingungen optimalen Partikelmenge übersteigt die Zunahme der absorbierten Lichtmenge die durch Zunahme der der Anbindung von markiertem Reagenz hinzugewonnene Chemilumineszenz (Fig. 1).

Der Einsatz von magnetisch abtrennbaren Partikel in einem ausreichend weiten Konzentrationsbereich ist deshalb nur durch das erfindungsgemäße Verfahren möglich.

Die in dieser Erfindung verwendeten Ablösereagenzien erfüllen folgende Anforderungen:
- Aufhebung mindestens einer intermolekularen Bindung, die im wesentlichen aus Wasserstoffbrücken sowie ionischen-, hydrophoben- und van der Waals Wechselwirkungen besteht.
- keine Beeinträchtigung der Chemilumineszenzreaktion durch Lichtabsorption
- keine Löschung des angeregten Zustandes des Luminophors
- keine Reaktion mit alkalischer H₂O₂-Lösung
- keine Beeinträchtigung der magnetischen Abtrennbarkeit der Magnetpartikel
- keine Auflösung oder Aggregation der Magnetpartikel.

Zu den Reagenzien, die diese Bedingungen erfüllen, zählen z. B. chaotrope Reagenzien, ionische Detergenzien, einige organische Lösungsmittel sowie Puffersubstanzen, die geeignet sind, einen pH-Wert im Bereich von 1.5 - 3 einzustellen. Neben den unten beispielhaft aufgeführten Reagenzien sind eine Vielzahl weiterer Reagenzien sowie ihre Kombinationen denkbar, die die angegeben Voraussetzungen erfüllen. In den Beispielen sind einige eingesetzt worden.

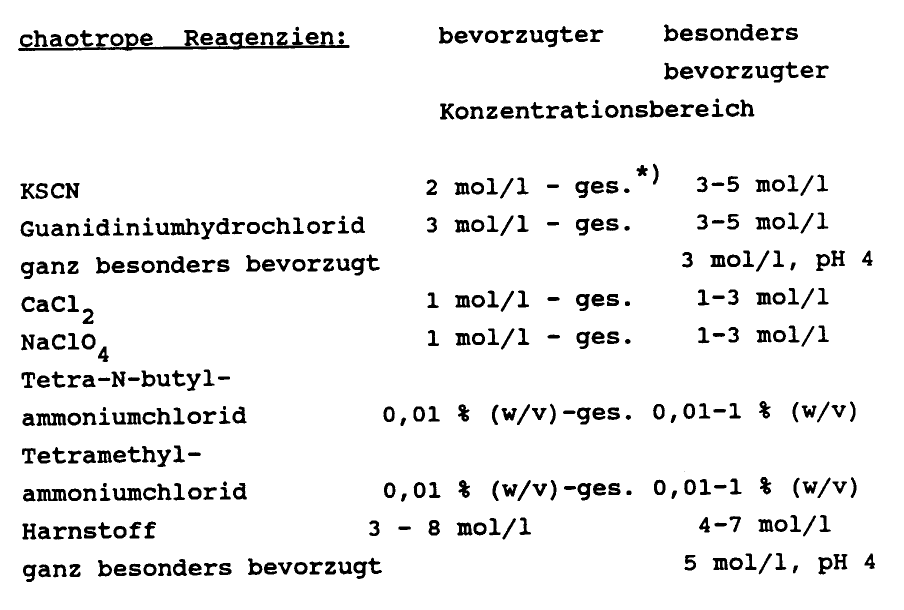

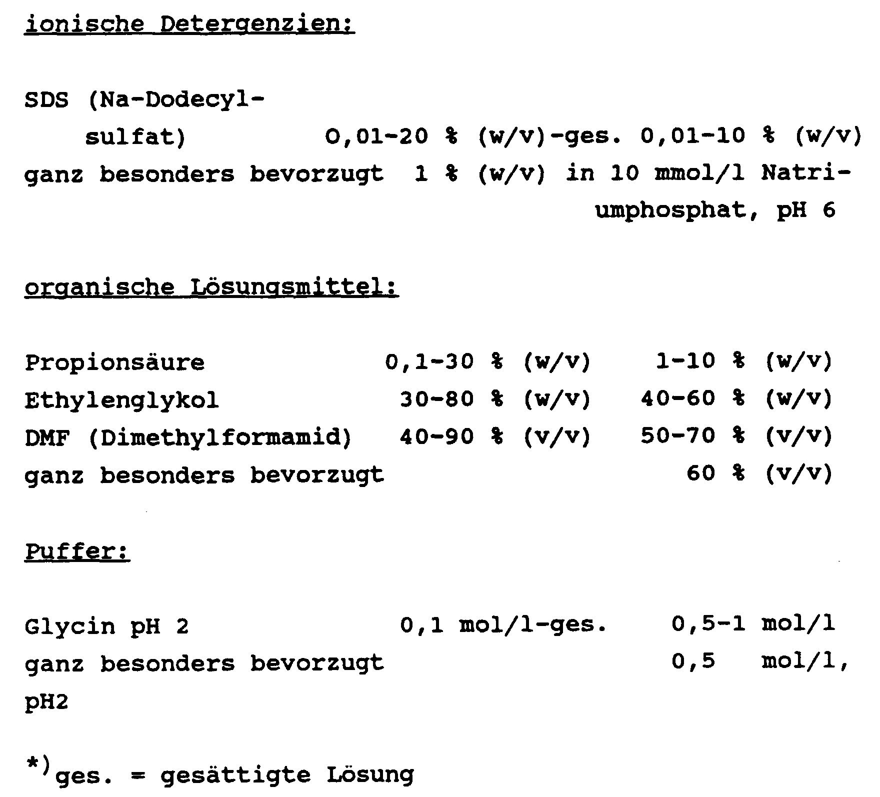

Diese Reagenzien können einzeln oder in Kombination eingesetzt werden.

Grundsätzlich ist es für den Fachmann leicht, die für eine bestimmte Substanz, bzw. Substanzkombination, optimalen Bedingungen durch wenige Versuche, bei denen er verschiedene Konzentrationen einsetzt, festzustellen.

Besonders bevorzugt sind die in den Beispielen angegebenen Ausführungsformen.

Die Erfindung ist grundsätzlich in Verfahren anwendbar, bei denen die Verteilung eines markierten Reagenzes zwischen einer Flüssigkeit und einer Festphase bestimmt wird.

Im Verlauf dieser Verfahren werden Festphase, Analyt und markiertes Reagenz entweder gleichzeitig oder in Folge eine geeignete Zeitdauer inkubiert. Nach der Inkubation wird die Festphase abgetrennt und dann in dem Ablösereagenz suspendiert und für eine kurze Zeit inkubiert. Die Partikel werden erneut abgetrennt und der Überstand gemessen.
Wenn eine Vielzahl von Proben gleichzeitig untersucht wird, lautet die Abfolge der Schritte typischerweise folgendermaßen:
A. Pipettieren von suspendierten, magnetisch anziehbaren Partikeln, Proben, Standards und markiertem Reagenz in Reaktionsgefäße.
B. Inkubation der Gefäße, die die Reaktionsmischung enthalten, bei der benötigten Temperatur für eine bestimmte Zeitdauer.
C. Abtrennen der magnetischen Partikel mit einer Anordnung von Magneten, die für die benutzten Gefäße geeignet ist.
D. Entfernen der Überstände durch Absaugen mit geeigneten Pipetten, Pumpen oder Vakuum.
E. Mehrmaliges Zugeben und Absaugen von Waschpuffer.
F. Entfernen bzw. Abschalten des zur Abtrennung der magnetisierbaren Partikel verwendeten Magnetfeldes und Resuspendieren der magnetischen Partikel im Ablösereagenz.
G. Abtrennen der Partikel mit dem Magnetsystem, Transferieren des Überstandes in ein geeignetes Gefäß und Messung der Markierung.

Die Ansprüche sind ein Bestandteil der Offenbarung.

Folgende Beispiele dienen zur Verdeutlichung der Erfindung, schränken sie aber in keiner Weise ein. Soweit nicht anders angegeben, werden als magnetisch abtrennbare Partikel Estapor^{R}-Partikel (0,35 µm) der Firma Rhone-Poulenc verwendet.

### Beispiel 1:

### Bestimmung von humanem Thyroid Stimulierendem Hormon (hTSH) gemäß dem Stand der Technik (Messung in Suspension)

Die folgenden Reagenzien wurden zusammen in Polystyrol-Röhrchen inkubiert:
- 0,006 mg; 0,013 mg; 0,019 mg; 0,025 mg; 0,050 mg; 0,075 mg; 0,1 mg und 0,125 mg magnetisch abtrennbare Partikel, die mit einem monoklonalem anti-hTSH-Antikörper beschichtet sind,
- 100 µl einer Lösung 50 µIU/ml hTSH in Human-Serum,
- 40 µl einer Lösung von einem mit einem Acridiniumderivat konjugierten, monoklonalen Antikörper,
- Suspendieren der Partikel,
- Inkubation der Suspension für 15 min bei 37°C,
- Abtrennen der Partikel mit einem Trennsystem der Fa. Gene-Trak und Absaugen des Überstandes,
- zu den abgetrennten Partikeln wird viermal 300 µl Waschpuffer gegeben und jeweils wieder abgesaugt,
- die Partikel werden dann in 100 µl Waschpuffer resuspendiert
- und in Polypropylen Röhrchen (1 ml) überführt. Diese Röhrchen werden in Polystyrol Röhrchen (5 ml) gesteckt. Mit einem Lumineszenzmeßgerät der Firma Berthold (Modell LB952T) wird durch Zugabe von 300 µl 0,1 N Salpetersäure und 0,5% H₂O₂ und 300 µl 0,25 N Natronlauge die Chemilumineszenz ausgelöst und gemessen (siehe Figur 1.).

### Beispiel 2:

### Bestimmung von humanem Thyroid Stimulierendem Hormon (hTSH)

Die folgenden Reagenzien wurden zusammen in Polystyrol-Röhrchen inkubiert:
- 0,025 mg; 0,125 mg und 0,250 mg magnetisch abtrennbare Partikel, die mit einem monoklonalem anti-hTSH-Antikörper beschichtet sind,
- 100 µl einer Lösung 50 µIU/ml hTSH in Human-Serum,
- 40 µl einer Lösung von einem mit einem Acridiniumderivat konjugierten monoklonalen Antikörper,
- Suspendieren der Partikel,
- Inkubation der Suspension für 15 min bei 37°C,
- Abtrennen der Partikel mit einem Trennsystem der Fa. Gene-Trak und Absaugen des Überstandes,
- zu den abgetrennten Partikeln wird viermal 300 µl Waschpuffer gegeben und jeweils wieder abgesaugt,
- die Partikel werden dann in 100 µl Abtrennreagenz resuspendiert, dann wird zur Messung
   a) die Suspension eingesetzt
   b) die abgelösten Magnetpartikel abgetrennt und der überstand eingesetzt
   c) die abgetrennten Magnetpartikel aus (b) in 100 µl Waschpuffer resuspendiert und diese Suspension eingesetzt
- die zur Messung eingesetzte Probe wird in Polypropylen-Röhrchen (1 ml) überführt. Die Röhrchen werden in Polystyrol-Röhrchen (5 ml) gesteckt. Mit einem Lumineszenzmeßgerät der Firma Berthold LB952T wird durch Zugabe von 300 µl 0,1 N Salpetersäure und 0,5% H₂O₂ und 300 µl 0,25 N Natronlauge die Chemilumineszenz ausgelöst und gemessen (siehe Tabelle 1).

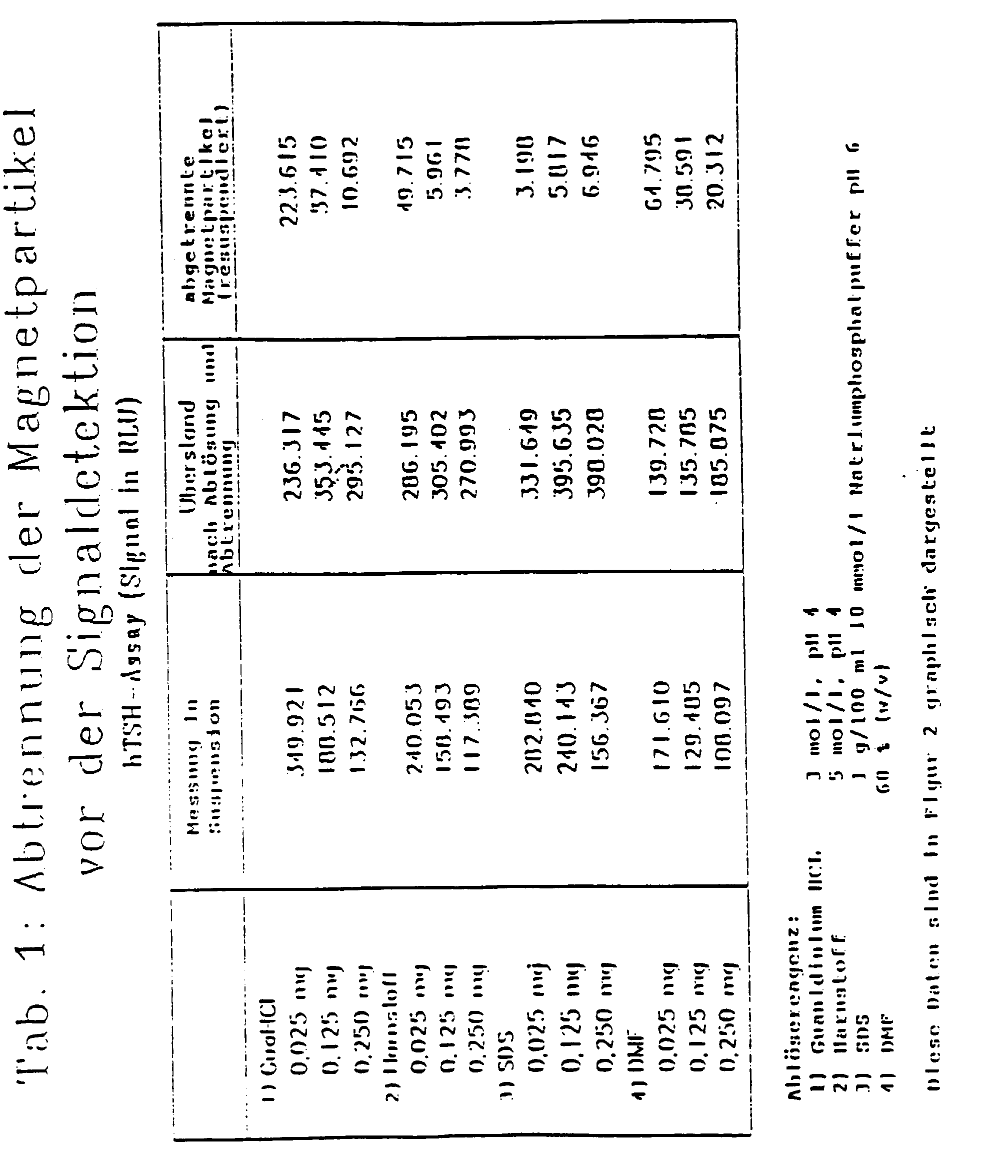

### Beispiel 3:

### Bestimmung von humanem Thyroid Stimulierendem Hormon (hTSH)

Die folgenden Reagenzien wurden zusammen in Flow-Röhrchen inkubiert:
- 0,125 mg magnetisch abtrennbare Partikel, die mit einem monoklonalen anti-hTSH-Antikörper beschichtet sind,
- 100 µl einer Standardlösung von hTSH in Human-Serum,
- 40 µl einer Lösung von einem mit einem Acridiniumderivat konjugierten monoklonalen Antikörper,
- Suspendieren der Partikel,
- Inkubation der Suspension für 15 min bei 37°C,
- Abtrennen der Partikel mit einem Trennsystem der Fa. Gene-Trak und Absaugen des Überstandes,
- zu den abgetrennten Partikeln wurden viermal 300 µl Waschpuffer gegeben,
- die Partikel wurden dann in in 100 µl Abtrennreagenz resuspendiert und anschließend 2 min in das Trennsystem gestellt,
- der überstand wird abgetrennt und in Flow-Röhrchen überführt. Die Flow-Röhrchen werden in Sarstedt-Röhrchen gesteckt. Mit einem Lumineszenzmeßgerät der Firma Berthold LB952T wird durch Zugabe von 300 µl 0,1 N Salpetersäure und 0,5 % H₂O₂ und 300 µl 0,25 N Natronlauge die Chemilumineszenz ausgelöst und gemessen.

**Tabelle 2:**

| Standard | | | Abtrennen der Partikel | kein Abtrennen der Partikel |
|---|---|---|---|---|
| | | | RLU | RLU |
| S0 | 0,0 | µIU/ml | 273 | 445 |
| S1 | 0,14 | µIU/ml | 1219 | 1149 |
| S2 | 0,47 | µIU/ml | 3179 | 2060 |
| S3 | 1,5 | µIU/ml | 9235 | 5621 |
| S4 | 4,8 | µIU/ml | 31477 | 17078 |
| S5 | 14,2 | µIU/ml | 96629 | 49972 |
| S6 | 50 | µIU/ml | 304099 | 164966 |
| S7 | 100 | µIU/ml | 496261 | 276073 |

Diese Daten sind in Figur 3 graphisch dargestellt.

## Patentansprüche

1. Heterogenes immunchemisches Verfahren zur Bestimmung eines Analyten in einem flüssigen Medium welches folgende Verfahrensschritte enthält:
a) Inkubation einer Probe, die den Analyten enthält mit einer partikulären Festphase auf der ein erster spezifischer Bindungspartner immobilisiert ist und einem zweiten spezifischen Bindungspartner in einer flüssigen Phase, der eine fluoreszente oder eine chemilumineszente Markierung trägt, wobei der zweite spezifische Bindungspartner zwischen der flüssigen Phase und der Festphase in Abhängigkeit von der Konzentration des Analyten verteilt wird,
b) mindestens einmalige Abtrennung der Festphase von der flüssigen Phase,
c) Resuspendierung der Festphase in einem flüssigen Reagenz A, welches geeignet ist, durch die Aufhebung mindestens einer intermolekularen Bindung - die im wesentlichen aus Wasserstoffbrücken, ionischen-, hydrophoben- und van der Waals Wechselwirkungen bestehen kann - die Markierung von der Festphase zu trennen, und welches ferner
i) die Lichtausbeute einer Chemilumineszenzreaktion nicht durch Lichtabsorption beeinträchtigt,
ii) den angeregten Zustand eines Luminophors nicht beeinträchtigt,
iii) nicht mit alkalischer H₂O₂ reagiert,
iv) nicht die magnetische Abtrennbarkeit von Magnetpartikeln beeinträchtigt und
v) nicht zu einer Auflösung oder Aggregation der Magnetpartikel führt;
d) Abtrennung der Festphase von der flüssigen Phase,
e) Bestimmung der Menge der von der Festphase abgelösten Markierung in der flüssigen Phase als Maß für die Menge des Analyten in der Probe.

2. Verfahren gemäß Anspruch 1, dadurch gekennzeichnet, daß die partikuläre Festphase magnetisch anziehbar ist und die Abtrennung der Festphase von der Flüssigphase durch magnetische Kraft erfolgt.

3. Verfahren gemäß Anspruch 1 oder 2, worin der zweite spezifische Bindungspartner eine chemilumineszente Markierung trägt.

4. Verfahren gemäß Anspruch 3, worin das Reagenz A Teil des zur Auslösung der Chemilumineszenz notwendigen Reagenzes ist.

5. Verfahren gemäß mindestens einem der Ansprüche 1 und 2, dadurch gekennzeichnet, daß das Reagenz A in einer wäßrigen Lösung eine oder eine Kombination aus mindestens zwei Substanzen der folgenden Reagenzienklasse enthält:
- chaotrope Reagenzien
- ionische Detergenzien
- organische Lösungsmittel
- Puffer im pH Bereich von 1.5 - 5

6. Verfahren gemäß mindestens einem der Ansprüche 1 und 2, dadurch gekennzeichnet, daß das Reagenz A in einer wäßrigen Lösung eines oder eine Kombination aus mindestens zwei Substanzen der folgenden Reagenzienklasse enthält:
- KSCN
- Guanidiniumhydrochlorid
- CaCl₂
- NaClO₄
- Tetra-N-butyl-ammoniumchlorid
- Tetramethyl-ammoniumchlorid
- Harnstoff
- SDS
- Propionsäure
- Ethylenglykol
- DMF
- Glycin im pH Bereich von 1,5 - 5

## Claims

1. A heterogeneous immunochemical method for determination of an analyte in a liquid medium, which comprises the following steps :
a) incubation of a sample which contains the analyte with a particulate solid phase on which a first specific binding partner is immobilized, and with a second specific binding partner which bears a fluorescent or a chemiluminescent label in a liquid phase, the second specific binding partner being partitioned between the liquid phase and the solid phase as a function of the concentration of the analyte,
b) removal at least once of the solid phase from the liquid phase,
c) resuspension of the solid phase in a liquid reagent A which is suitable for separating, by abolishing at least one intermolecular binding
- which can consist essentially of hydrogen bonds, ionic, hydrophobic and van der Waals interactions - the label from the solid phase, and which furthermore
i) does not impair the light yield of a chemiluminescence reaction by light absorption,
ii) does not impair the excited state of a luminophore,
iii) does not react with alkaline H₂O₂,
iv) does not impair the magnetic removability of magnetic particles, and
v) does not lead to dissolution or aggregation of the magnetic particles;
d) removal of the solid phase from the liquid phase,
e) determination of the amount of the label detached from the solid phase in the liquid phase as a measure of the amount of the analyte in the sample.

2. The method as claimed in claim 1, wherein the particulate solid phase is magnetically attractable, and the removal of the solid phase from the liquid phase takes place by magnetic force.

3. The method as claimed in claim 1 or 2, wherein the second specific binding partner bears a chemiluminescent label.

4. The method as claimed in claim 3, wherein reagent A is part of the reagent needed to elicit the chemiluminescence.

5. The method as claimed in at least one of claims 1 and 2, wherein reagent A contains in an aqueous solution one or a combination of at least two substances of the following class of reagents:
- chaotropic reagents
- ionic detergents
- organic solvents
- buffers in the pH range 1.5 - 5.

6. The method as claimed in at least one of claims 1 and 2, wherein reagent A contains in an aqueous solution one or a combination of at least two substances of the following class of reagents:
- KSCN
- guanidinium hydrochloride
- CaCl₂
- NaClO₄
- tetra-N-butyl-ammonium chloride
- tetramethyl-ammonium chloride
- urea
- SDS
- propionic acid
- ethylene glycol
- DMF
- glycine in the pH range 1.5 - 5.

## Revendications

1. Procédé immunochimique hétérogène pour la détermination d'un corps à analyser dans un milieu liquide, comportant les étapes suivantes :
a) incubation d'un échantillon qui contient un corps à analyser, avec une phase solide particulaire sur laquelle un premier partenaire de liaison spécifique est immobilisé, et avec un second partenaire de liaison spécifique, dans une phase liquide, qui porte un marqueur fluorescent ou un marqueur chimiluminescent, le second partenaire de liaison spécifique étant réparti entre la phase liquide et la phase solide, en fonction de la concentration du corps à analyser,
b) séparation au moins une fois de la phase solide d'avec la phase liquide,
c) remise en suspension de la phase solide dans un réactif liquide A qui est approprié à séparer le marqueur d'avec la phase solide, par la suppression d'au moins une liaison intermoléculaire - pouvant consister essentiellement en ponts hydrogène, interactions ioniques, hydrophobes et de van der Waals - et qui en outre
I) ne nuit pas, par absorption de lumière, au rendement lumineux d'une réaction de chimiluminescence,
II) ne nuit pas à l'état excité d'un luminophore,
III) ne réagit pas avec H₂O₂ en solution alcaline,
IV) ne nuit pas à l'aptitude de particules magnétiques à la séparation magnétique et
V) n'entraîne pas une dissolution ni une agrégation des particules magnétiques,
d) séparation de la phase solide d'avec la phase liquide,
e) détermination de la quantité du marqueur détaché de la phase solide, dans la phase liquide, en tant que mesure de la quantité du corps à analyser dans l'échantillon.

2. Procédé selon la revendication 1, caractérisé en ce que la phase solide est apte à l'attraction magnétique, et la séparation de la phase solide d'avec la phase liquide s'effectue sous l'effet d'une force magnétique.

3. Procédé selon la revendication 1 ou 2, dans lequel le second partenaire de liaison spécifique porte un marqueur chimiluminescent.

4. Procédé selon la revendication 3, dans lequel le réactif A fait partie du réactif requis pour le déclenchement de la chimiluminescence.

5. Procédé selon au moins l'une des revendications 1 et 2, caractérisé en ce que le réactif A contient, dans une solution aqueuse, une substance ou une association d'au moins deux substances appartenant à la classe de réactifs suivante:
- réactifs chaotropes,
- détergents ioniques,
- solvants organiques,
- tampons dans la plage de pH allant de 1,5 à 5.

6. Procédé selon au moins l'une des revendications 1 et 2, caractérisé en ce que le réactif A contient, dans une solution aqueuse, une substance ou une association d'au moins deux substances appartenant à la classe de réactifs suivante:
- KSCN,
- chlorhydrate de guanidinium,
- CaCl₂,
- NaClO₄,
- chlorure de tétra-N-butylammonium,
- chlorure de tétraméthylammonium,
- urée,
- SDS,
- acide propionique,
- éthylèneglycol,
- DMF,
- glycine,
dans la plage de pH allant de 1,5 à 5.
